(19) Europäisches Patentamt
European Patent Office
Office européen des brevets

(11) EP 2 457 591 A2

(12) # EUROPEAN PATENT APPLICATION

published in accordance with Art. 153(4) EPC

(43) Date of publication:
**30.05.2012 Bulletin 2012/22**

(21) Application number: **10801996.9**

(22) Date of filing: **22.07.2010**

(51) Int Cl.:
***A61K 47/34*** *(2006.01)* ***A61K 9/51*** *(2006.01)*
***A61K 9/127*** *(2006.01)* ***A61K 9/107*** *(2006.01)*

(86) International application number:
**PCT/ES2010/070504**

(87) International publication number:
**WO 2011/009991 (27.01.2011 Gazette 2011/04)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **24.07.2009 ES 200901645**

(71) Applicant: **Universidade De Santiago De Compostela**
**15782 Santiago de Compostela (ES)**

(72) Inventors:
- **SOUSA HERVES, Ana**
  **(ES)**
- **FERNANDEZ MEGIA, Eduardo**
  **(ES)**
- **RIGUERA VEGA, Ricardo**
  **(ES)**

(74) Representative: **Carvajal y Urquijo, Isabel et al**
**Clarke, Modet & C.**
**C/ Goya, 11**
**28001 Madrid (ES)**

(54) **DENDRITIC PIC MICELLES WITH BIOACTIVE PROTEINS**

(57) Dendritic PIC micelles with bioactive proteins. The invention relates to a Polyion Complex (PIC) polymeric micelle formed by interaction between a bioactive protein and a dendritic block copolymer of opposite charge. The conventional low stability of PIC micelles with bioactive proteins vis-à-vis ionic strength is offset in the present invention by the stability provided by the dendritic block. The overall process for preparing these micelles is facilitated by the simplicity of the drendritic block copolymer synthesis.

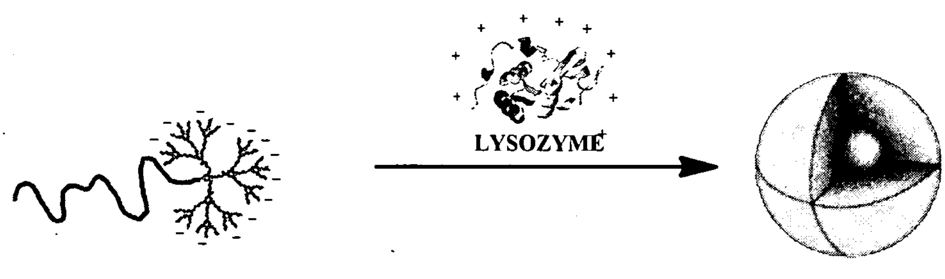


Figure 1



Printed by Jouve, 75001 PARIS (FR)

## Description

## Field of the Art

The present invention refers to a dendritic polyion complex (PIC) micelle which comprises a bioactive protein as one of the polyionic blocks which form the micelle. More specifically, the present invention refers to a PIC micelle formed by the electrostatic interaction between a dendritic block copolymer and a bioactive protein with the opposite charge. This micelle can be stable under physiological conditions and has applications in nanomedicine, medical and biological engineering, protein and drug delivery processes, biotechnology, therapy and diagnostics.

## Background of the Invention

The use of bioactive proteins as therapeutic agents has attracted a great deal of interest over the past few decades due to their high and selective therapeutic activity. Thus, for example, various diseases caused by a deficiency of certain lysosomal enzymes can only be treated by the administration of exogenous enzymes [Enzymes as Drugs 1981, p. 167, J. Wiley]. Insulin, a peptide hormone, is probably the most widely used of the many therapeutic proteins available, although anti-tumoural enzymes (which act by destroying specific amino acid sequences required for tumour growth), digestive enzymes (for the treatment of various digestive tract disorders) and antibacterial, antiviral and antiinflammatory enzymes are also becoming increasingly important.

Despite this, the use of proteins as therapeutic agents has two major drawbacks, namely their slow and inefficient transport through biological barriers and their short *in vivo* half-life as a result of enzymatic degradation and metabolism. The incorporation of bioactive proteins into delivery systems (nanoparticles, liposomes, etc.) presents numerous advantages with respect to more conventional administration pathways. Thus, the solubility, the stability of these therapeutic agents against enzymatic degradation and their pharmacokinetics have been improved, and in some cases controlled release of the therapeutic protein has been achieved. However, the efficacy of these systems *in vivo* has yet to be demonstrated due to various factors, including, in the case of liposomes, their limited stability in biological media [J. Polym. Sci. Part A: Polym. Chem. 2008, 46, 1]. One of the main problems encountered when designing a suitable transport system for bioactive proteins is their loss of activity. Thus, as a result of the tendency of proteins to denature, the preparation, purification and storage conditions are somewhat limited. Furthermore, the chosen transport system must be biocompatible and should protect the protein against opsonisation by the reticuloendothelial system (RES).

In this context, Polyion Complex (PIC) micelles appear to be a promising transport vehicle for bioactive proteins by allowing their incorporation via non-covalent bonds under physiological conditions, thereby preserving their activity. Furthermore, such micelles possess a shell formed from a hydrophilic biocompatible polymer [normally poly(ethylene glycol), PEG] which, once in the bloodstream, prevents recognition and opsonisation by the immune system, thereby resulting in longer circulation times [Pharm. Res. 2001, 18, 1411].

PIC micelles [Macromolecules 1995, 28, 5294. Bioconjugate Chem. 1995, 6, 639] are a type of polymeric micelle formed by the electrostatic interaction between oppositely charged polymers or macromolecules in which one of the species is a block copolymer containing a hydrophilic and biocompatible block. They are nanostructures with a very low polydispersity and a size similar to that of viruses or lipoproteins (10-200 nm), thus facilitating their entry into cells. A further advantage of PIC micelles is their tendency to passively accumulate in solid tumours as a result of the Enhanced Permeability and Retention (EPR) effect [Cancer Res. 1986, 46, 6387], which, together with their prolonged circulation times in the bloodstream, results in their selective accumulation in tumour tissues.

The first example of a PIC micelle formed by the electrostatic interaction between a polyionic polymer and a protein was described by Kataoka and co-workers [Macromolecules 1998, 31, 288]. Indeed, these authors reported both conservation of the enzyme activity of various proteins inside the PIC micelle and an increased activity in some cases due to the specific properties of the micellar core [J. Am. Chem. Soc. 2003, 125, 15306]. In this sense, it has been proposed that protein-containing PIC micelles can be considered as nano-reactors with applications in the fields of medical and biological engineering.

However, the main drawback of PIC micelles formed from proteins is their low stability with respect to the ionic strength, which means that their application in biological systems is severely limited. The stability of PIC micelles towards saline concentrations is directly related to the charge density of their components. Thus, PIC micelles have been reported to be stable under physiological conditions when their constituent blocks have a high charge density, as is the case of poly(amino acids). However, replacement of a poly(amino acid) with a bioactive protein results in the immediate dissociation of the PIC micelles under physiological conditions. This finding has been explained on the basis of the low charge density of such proteins. Thus, whereas a commercial poly(amino acid) such as Poly-L-lysine contains a positive charge approximately every 200 Da, a protein such as lysozyme only has such a charge every 2000 Da [Angew. Chem. Int. Ed. 2009, DOI: 10.1002/anie.200900064].

A marked increase in the stability of PIC micelles has been achieved recently by using dendrimers [Macromolecules 2003, 36, 1304] and dendritic block copolymers [Chem. Commun. 2008, 27, 3136], with this increase being attributed mainly to the greater rigidity associated with this type of structure. PIC micelles formed from dendritic block copolymers have the additional advantage that the latter are easy to synthesise, thus making them cheap and therefore ideal from an industrial viewpoint [Chem. Comm. 2008, 27, 3136].

In accordance with the prior known and established state-of-the-art, PIC micelles formed from dendritic block copolymers and bioactive proteins are currently unknown.

As such, the present invention shows that PIC micelles containing dendritic block copolymers are an optimal delivery system for bioactive proteins that overcomes the current problems in the state-of-the-art.

## Description of the invention

### Definitions

Dendrimer: A highly branched polymer in which the repeating units are organised into generations from a single focal point. Their nanometric size and multivalency mean that these macromolecules are ideal for use in receptor-ligand interaction processes.

Block copolymer: A copolymer is a polymer formed from two or more monomeric species. Those copolymers formed from two blocks of different polymerised monomers are known as block copolymers.

Bioactive proteins: Macromolecules formed from linear chains of amino acids that regulate or are involved in a biological process. Bioactive proteins are characterised by their ability to organise themselves in four structural levels known as the primary, secondary, tertiary and quaternary structure. The primary structure is a description of the covalent bonds that join the various amino acids in a protein chain. The secondary structure refers to particularly stable arrangements of the amino acids that lead to repetitive structural patterns. The tertiary structure describes all aspects of the three-dimensional folding of the protein. Finally, when a protein possesses two or more polypeptide sub-units, their spatial arrangement is known as the quaternary structure. The bioactivity of a protein depends on these four organisational levels. Thus, if the three-dimensional structure of a protein is destroyed (denaturation), its function is also destroyed.

Bioactive proteins are also characterised by their low charge density as only five of the 20 proteinaceous amino acids contain ionisable groups.

Isoelectric point (pI): The isoelectric point is the pH at which an amphoteric substance has zero nett charge. This concept is particularly important for bioactive proteins as they are practically insoluble at their isoelectric point. Once the isoelectric point is known, the nett charge of a protein can be varied by varying the pH. Thus, a protein with a high isoelectric point such as lysozyme (pI~11) will have a nett positive charge in aqueous solution at a pH below 11 and a nett zero charge at pH 11.

PIC micelles: A type of polymeric micelle characterised in that their formation is mainly based in electrostatic interactions between oppositely charged polymers or macromolecules. They are unusual in that the ratio between their charges is stoichiometric, thus meaning they have a nett zero charge. They were first described by Kataoka (1995), who named them "Polyion Complex Micelles" (PIC Micelles).

Enhanced Permeability and Retention (EPR) effect: An effect by which macromolecules (for example polymeric micelles) tend to passively accumulate in tumour tissues. This fact has been used in the context of anticancer therapy in order to selectively accumulate drug toxicity in cancerous tissues whilst minimising the effects in healthy tissues.

It is known that this effect occurs as a result of two main factors, namely (i) the hyperpermeability of the tumour vasculature, which allows the transport of macromolecules to the tumour, and (ii) poor lymphatic drainage, which results in high retention of macromolecules in the tumour.

In order to overcome the problems encountered in the prior state-of-the-art, the present invention provides the following improvements.

The present invention provides a type of PIC micelle formed by the electrostatic interaction between a charged dendritic block copolymer and a bioactive protein.

A key advantage of the present invention is the proposed solution to the problem of the instability of PIC micelles containing bioactive proteins under physiological saline conditions, which involves the incorporation of charged dendritic block copolymers. In this manner, the low stability with respect to the ionic force resulting from the low charge density of bioactive proteins is compensated by the greater stability provided by the dendritic block. The present invention is the first example of a dendritic PIC micelle containing bioactive proteins that is stable under physiological conditions. This increased stability is reflected by the ability to lyophilise these micelles, thereby facilitating their conservation and storage.

A further key advantage offered by the present invention is that, in contrast to other protein delivery systems, the bioactive protein itself is one of the constituents of the micelle. This means that a system in which the protein is subsequently encapsulated need not be prepared.

Another key advantage of the present invention is that the bioactive protein-containing PIC micelles are formed in an aqueous medium, with no need for covalent bond formation, under very mild conditions, thus meaning that the

activity of the protein should be unaffected.

Finally, it should be noted that the overall preparation process for the bioactive protein-containing PIC micelles described in the present invention is extremely simple due to the facile synthesis of the dendritic block copolymers, thus meaning that this is a cheap, and therefore ideal, process from an industrial viewpoint.

The present invention refers to a polymeric micelle formed by the electrostatic interaction between:

a) a dendritic block copolymer represented by the general formula [1];

$$\mathbf{P\,D\,q\,(c)}\quad [1]$$

where **P** is a polymer, **D** is a dendritic structure, **q** represents the number of charged atoms at the periphery of the dendritic structure, which varies between 1 and 5000, **c** represents a positive or negative charge, and

b) a bioactive protein **A** with a nett charge **c'** opposite to **c.**

The present invention also relates to a polymeric micelle **characterized in that** the polymer **P** defined in claim 1 is preferably selected from linear polymers, block copolymers, copolymers, terpolymers, graft copolymers, graft terpolymers and amphiphilic copolymers.

The present invention also relates to a polymeric micelle **characterized in that** the polymer **P** is preferably selected from natural polymers or polymers produced by chemical synthesis or biotechnological processes.

In a particular embodiment, the polymer **P** is preferably selected from N-(2-hydroxypropyl)methacrylamide (HPMA), poly(styrene-co-maleic acid/anhydride) (SMA), poly(divinyl ether maleic anhydride) (DIVEMA), poly(N-vinyl pyrrolidone) (PVP), poly(N-acryloyl)morpholine (PAcM), poly(ethylene glycol) (PEG), poly(propylene oxide) (POP) and their derivatives.

In another particular embodiment, the polymer **P** is poly(ethylene glycol) (PEG).

The present invention also relates to a polymeric micelle **characterized in that** the bioactive protein **A** is preferably selected from antibodies, antibody fragments, heteroproteins, enzymes and hormones.

In a particular embodiment, the bioactive protein **A** is preferably selected from myoglobin, lysozyme and insulin.

In a preferred embodiment, dendritic structure **D** is a dendrimer, dendron or dendritic polymer constructed from one or more repeating units.

The present invention also relates to a polymeric micelle **characterized in that** the dendritic structure **D** is preferably a dendron constructed from a repeating unit represented by general formula [2]

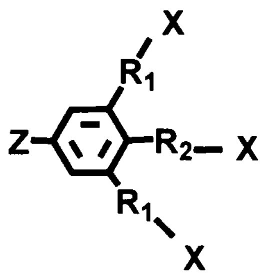

[2]

wherein **Z** represents a covalent bond between the repeating unit and the polymer **P** or the repeating unit and the previous generation,

$\mathbf{R_1}$ and $\mathbf{R_2}$ are linear or branched chains which can be identical or different and which are characterized by containing alkyl, alcohol, thiol, azide, nitrile, amine, imide, imine, cyanate, isocyanate, isothiocyanate, ether, thioether, ketone, aldehyde, ester, carboxylic acid or aromatic groups;

**X** represents the repeating unit of the following generation or alternatively an anionic or cationic group.

In another particular embodiment, the dendritic structure **D** is preferably a dendron constructed from a repeating unit represented by general formula [2], wherein

**Z** is preferably an amide bond;

$\mathbf{R_1}$ and $\mathbf{R_2}$ are identical and selected from poly(ethylene glycol) or oligo(ethylene glycol) chains, they are preferably

triethylene glycol;

when **X** is an anionic group it is preferably selected from carboxylic acids and their derivatives, sulfates and their derivatives, sulfonates and their derivatives, phosphates and their derivatives, phosphonates and their derivatives, arylphosphonic acids and their derivatives, phenols and their derivatives;

when **X** is a cationic group it is preferably selected from amines, polyamines, oligoamines, preferably spermidines, spermines, anilines, benzylamines, imidazoles, morpholines, ammonium salts, primary, secondary or tertiary amines or guanidinium groups.

A specific aspect of the invention relates to a polymeric micelle as defined above formed by the electrostatic interaction between a PEG-dendritic block copolymer with benzoate groups at its periphery and the bioactive protein lysozyme, as shown schematically in Figure 1.

Another specific aspect, the invention relates to a polymeric micelle as defined above formed by the electrostatic interaction between a PEG-dendritic block copolymer with benzoate groups at its periphery and the bioactive protein myoglobin.

Another specific aspect, the invention relates to a polymeric micelle as defined above formed by the electrostatic interaction between a PEG-dendritic block copolymer with positively charged aniline groups at its periphery and the bioactive protein insulin.

The present invention also refers to the encapsulation of a drug, diagnostic agent, molecule or macromolecule in the interior of a polymeric micelle as defined above.

A further aspect of the present invention refers to the use of a micelle as defined above as a carrier for the therapeutic administration of a bioactive protein.

In a further aspect, the present invention refers to the use of a micelle as defined above as a carrier for administering of a drug or diagnostic agent, molecule or macromolecule. Another aspect of the present invention refers to a pharmaceutical composition comprising a polymeric micelle as defined above and an active ingredient.

As described in detail, the present invention provides a type of polymeric micelle formed from bioactive proteins which can be stable under physiological conditions and is prepared following a fast, simple and cheap method. The type of micelle described can be used in nanomedicine, medical and biological engineering, protein and drug delivery processes, biotechnology, therapy and diagnostics.

**Description of the figures**

Figure 1. Formation of a lysozyme-containing PIC micelle

Figure 2. Formation of a lysozyme-containing PIC micelle and the DLS histogram.

Figure 3. AFM image of a lysozyme-containing PIC micelle formed from dendritic block copolymers with benzoate groups.

Figure 4. Formation of a myoglobin-containing PIC micelle and the DLS histogram.

Figure 5. Formation of an insulin-containing PIC micelle and the DLS histogram.

Figure 6. AFM image of an insulin-containing PIC micelle formed from dendritic block copolymers with positively charged aniline groups.

Preparation of the bioactive protein-containing micelles of this invention is illustrated by way of the following examples, which should nevertheless not be considered to limit the scope of said invention:

**EXAMPLE 1.** Synthesis of block copolymers.

The dendritic unit **2** was prepared from methyl gallate **1** as shown in **Scheme 1.** The first-generation block copolymer **PEG-[G1]-$N_3$** was obtained by coupling dendritic unit **2** to PEG-$NH_2$ **(3).**

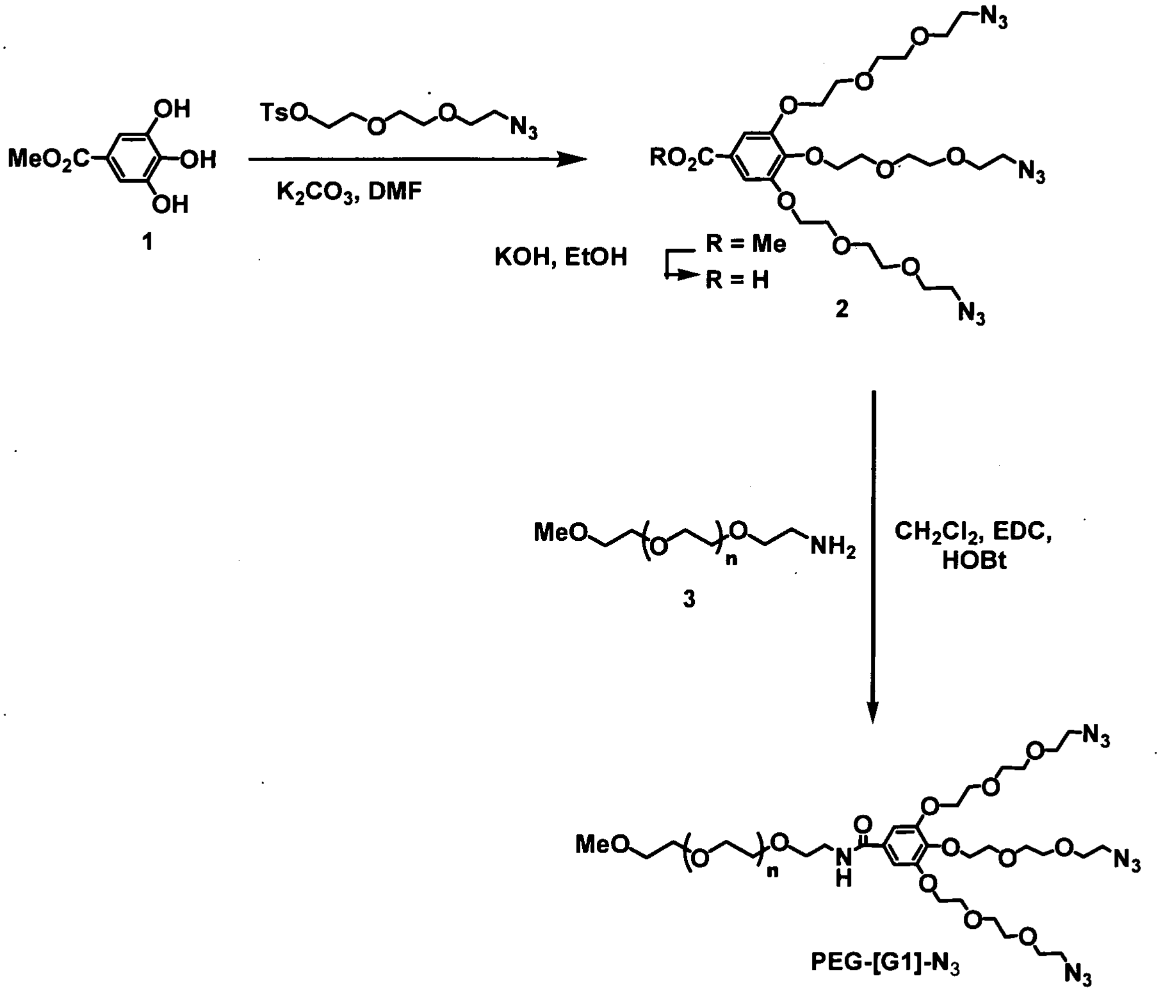

1
2
3
PEG-[G1]-N$_3$

**Scheme 1**

Higher generation block copolymers were obtained by reduction of the terminal azide groups of the previous generation by catalytic hydrogenation and coupling of the resulting amines to dendritic unit **2** as shown in **Scheme 2.**

PEG-[G1]-$N_3$

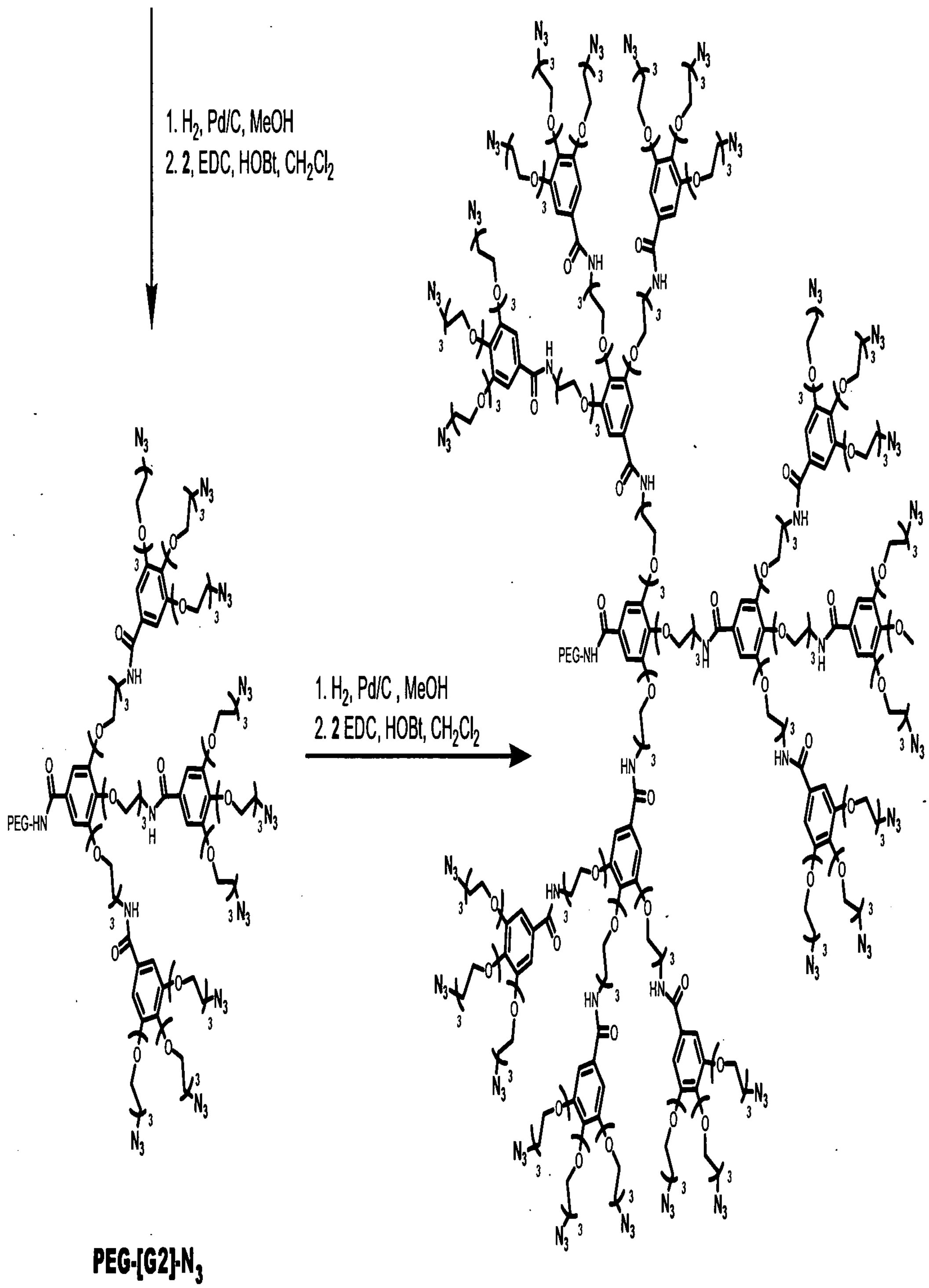


PEG-[G2]-$N_3$

PEG-[G3]-$N_3$

**Scheme 2**

**EXAMPLE 2.** General procedure for the anionic functionalisation of the PEG-dendritic block copolymers **(PEG-[Gn]-$N_3$).**

Functionalisation of the block copolymers with anionic groups was performed by a Cu(I)-catalysed azide-alkyne [3+2] cycloaddition reaction. This means that the anionic ligands to be introduced must be functionalised with terminal alkyne groups.

The PEG-dendritic block copolymer **PEG-[Gn]-$N_3$** and the anionic ligand were dissolved in a mixture of *t*-BuOH and $H_2O$ (1:1). Catalytic amounts of $CuSO_4$ and sodium ascorbate were then added **(Scheme 3).**

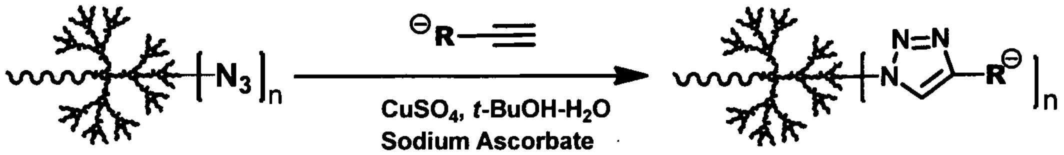


**PEG-[$G_n$]-$N_3$**

**Scheme 3**

**EXAMPLE 3.** General procedure for the cationic functionalisation of the PEG-dendritic block copolymers **(PEG-[Gn]-$N_3$).**

Two strategies can be used to introduce cationic groups at the periphery of the block copolymer:

1. Reduction of the terminal azides, for example by catalytic hydrogenation in an acidic medium, leads to ammonium salts of the corresponding primary amines at the periphery of the dendrimer. The formation of **PEG-[G3]-$NH_3^+$** is shown in **Scheme 4.**
2. A Cu(I)-catalysed azide-alkyne [3+2] cycloaddition reaction, as described in **Scheme 3,** but using cationic ligands functionalised with terminal alkynes.

PEG-[G3]-$N_3$

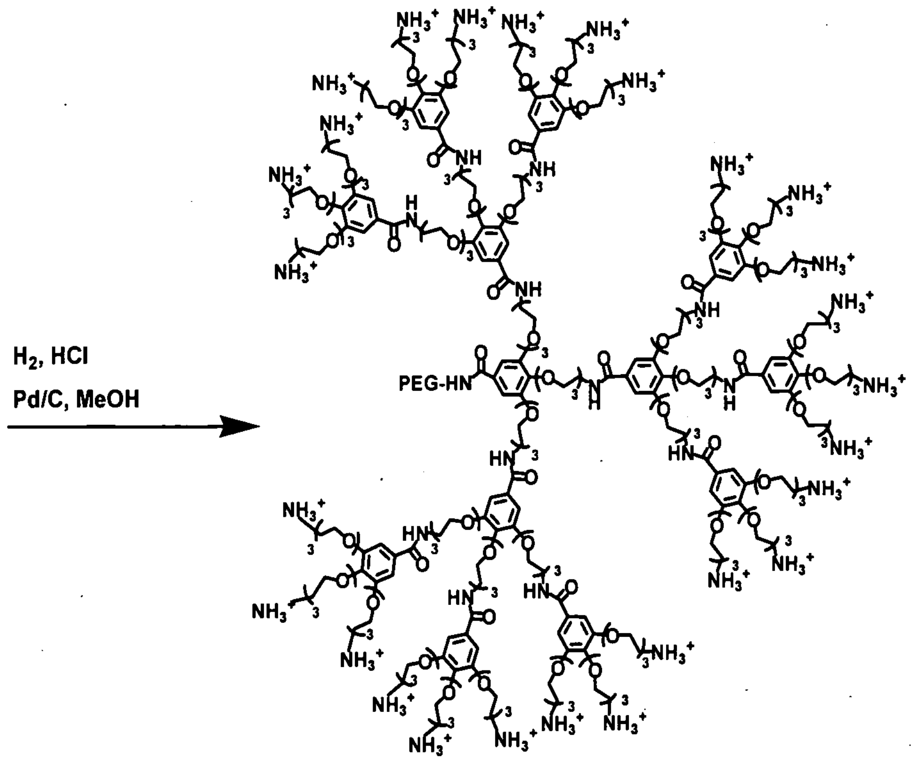


PEG-[G3]-$NH_3^+$

**Scheme 4**

**EXAMPLE 4.** Preparation of PIC micelles from a block copolymer functionalised with terminal benzoate groups and the bioactive protein lysozyme (pI > 7).

The block copolymer **PEG-[G3]-Benzoate,** obtained from PEG-$NH_2$ ($M_n$ 5219) following the procedure described in examples **1** and **2,** was dissolved in phosphate buffer (PBS) at pH 7.4. The protein lysozyme (commercial product) was dissolved in the same pH 7.4 PBS buffer in a separate recipient. The two solutions were then filtered and mixed, in a stoichiometric charge ratio, considering only the positive charges on the protein at the working pH for this purpose, to produce PIC micelles in a final solution with pH 7.4. After stirring magnetically for 24 h, the resulting solution was filtered and analysed by dynamic light scattering (DLS). The DLS measurements confirmed the presence of polymeric micelles with a very low polydispersity index (0.1) and a size of around 120 nm. (Figure 2). This size was confirmed by atomic force microscopy (AFM). To determine the stability of the lysozyme-containing PIC micelles under physiological conditions, NaCl was added to the original micelle solution to a concentration of 150 mM, the mixture heated to 37 °C and the resulting micelle solution subsequently analysed by DLS. No change in size or any other signs of destabilisation were observed. Characterisation. In order to visualise the dendritic lysozyme-containing PIC micelles by AFM, an aliquot of a micelle solution with a concentration of 1.18 mg/mL in 10 mM PBS, pH 7.4, was removed and diluted with Milli-Q water to a concentration of 0.05 mg/mL. A 10-μL portion of this solution was deposited on a silicon surface and allowed to dry at room temperature. Spherical particles with a diameter of approximately 140 nm, in good agreement with the DLS measurements, were observed (Fig. 3).

Micelle stability. The dendritic lysozyme-containing PIC micelles are stable for more than a month at 4 °C in 10 mM PBS, pH 7.4, and for several weeks at this temperature in the presence of 150 mM NaCl. Furthermore, these micelles are stable for several weeks at room temperature in PBS 10 mM, pH 7.4, although storage at this temperature is not recommended due to their proteinaceous nature.

Further proof of the stability of these micelle arises form the fact that they can be lyophilised and subsequently re-suspended in the same volume, with only a small decrease in their size (by DLS).

**EXAMPLE 5.** Preparation of PIC micelles from a block copolymer functionalised with terminal benzoate groups and the bioactive protein myoglobin (pI ~ 7).

The block copolymer **PEG-[G3]-Benzoate,** obtained from $PEG-NH_2$ ($M_n$ 5219) following the procedure described in examples 1 and 2, was dissolved in phosphate buffer (PBS) at pH 9. The myoglobin (commercial product) was dissolved in PBS pH 3 in a separate recipient. The two solutions were then filtered and mixed, in a stoichiometric charge ratio, considering only the positive charges on the protein at the working pH for this purpose, to produce PIC micelles at a final pH of 7.4. After stirring magnetically for 24 h, the resulting solution was filtered and analysed by DLS (Figure 4). The DLS measurements confirmed the presence of polymeric micelles with a low polydispersity index (0.15) and a size of around 50 nm. These values were confirmed by AFM.

**EXAMPLE 6.** Preparation of PIC micelles from a block copolymer functionalised with positively charged aniline groups and the bioactive protein insulin (pI < 7).

The block copolymer **PEG-[G3]-Aniline HCl,** obtained from $PEG-NH_2$ ($M_n$ 5219) following the procedure described in examples **1** and **3,** was dissolved in phosphate buffer (PBS) at pH 3. The insulin (commercial product) was dissolved in PBS pH 12 in a separate recipient. The two solutions were then filtered and mixed, in a stoichiometric charge ratio, considering only the positive charges on the protein at the working pH for this purpose, to produce PIC micelles at a final pH of 7.4. After stirring magnetically for 24 h, the resulting solution was filtered and analysed by DLS (Figure 5). The DLS measurements confirmed the presence of polymeric micelles with a low polydispersity index (0.15) and a size of around 45 nm. These values were confirmed by AFM.

In order to visualise the dendritic insulin-containing PIC micelles by AFM, an aliquot of a micelle solution with a concentration of 0.96 mg/mL in 10 mM PBS, pH 7.4, was removed and diluted with Milli-Q water to a concentration of 0.05 mg/mL. A 10-μL portion of this solution was deposited on a silicon surface and allowed to dry at room temperature. Spherical particles with a diameter of approximately 45 nm, in good agreement with the DLS measurements, were observed (Fig. 6).

To determine the stability of the insulin-containing PIC micelles under physiological conditions, NaCl was added to the original micelle solution to a concentration of 150 mM, the mixture heated to 37 °C and the resulting micelle solution subsequently analysed by DLS. No change in size or any other signs of destabilisation were observed. The dendritic insulin-containing PIC micelles are stable for several weeks at room temperature, although storage at this temperature is not recommended due to their proteinaceous nature. Furthermore, the micelles can be stored for more than a month at 4 °C in either 10 mM PBS, pH 7.4, or 10 mM PBS, pH 7.4, plus 150 mM NaCl.

## Claims

**1.** A polymeric micelle formed by the electrostatic interaction between:

a) a dendritic block copolymer represented by the general formula [1];

$$\mathbf{P\,D\,q\,(c)} \quad [1]$$

where **P** is a polymer, **D** is a dendritic structure, **q** represents the number of charged atoms at the periphery of the dendritic structure, which varies between 1 and 5000, **c** represents a positive or negative charge, and
b) a bioactive protein **A** with a nett charge **c'** opposite to **c.**

**2.** A polymeric micelle according to claim 1 wherein the polymer **P** is preferably selected from linear polymers, block copolymers, copolymers, terpolymers, graft copolymers, graft terpolymers and amphiphilic copolymers.

**3.** A polymeric micelle according to claims 1 and 2 wherein the polymer **P** is preferably selected from natural polymers or polymers produced by chemical synthesis or biotechnological processes.

**4.** A polymeric micelle according to claims 1, 2 and 3 wherein the polymer **P** is preferably selected from N-(2-hydroxypropyl)methacrylamide (HPMA), poly(styrene-co-maleic acid/anhydride) (SMA), poly(divinyl ether maleic anhydride) (DIVEMA), poly(N-vinyl pyrrolidone) (PVP), poly(N-acryloyl)morpholine (PAcM), poly(ethylene glycol) (PEG), poly(propylene oxide) (POP) and their derivatives.

5. A polymeric micelle according to the previous claims wherein the polymer **P** is poly(ethylene glycol) (PEG).

6. A polymeric micelle according to claim 1 wherein the bioactive protein **A** is preferably selected from antibodies, antibody fragments, heteroproteins, enzymes and hormones.

7. A polymeric micelle according to claims 1 and 6 wherein the bioactive protein **A** is preferably selected from myoglobin, lysozyme and insulin.

8. A polymeric micelle according to claim 1 wherein the dendritic structure **D** is a dendrimer, dendron or dendritic polymer constructed from the same or different repeating units.

9. A polymeric micelle according to claims 1 and 8 wherein the dendritic structure **D** is preferably a dendron constructed from a repeating unit represented by general formula [2].

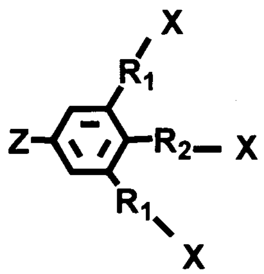

[2]

wherein **Z** represents a covalent bond between the repeating unit and the polymer **P** or the repeating unit and the previous generation,
$R_1$ and $R_2$ are linear or branched chains which can be identical or different and are **characterized by** containing alkyl, alcohol, thiol, azide, nitrile, amine, imide, imine, cyanate, isocyanate, isothiocyanate, ether, thioether, ketone, aldehyde, ester, carboxylic acid or aromatic groups;
**X** represents the repeating unit of the following generation or alternatively an **anionic** or cationic group.

10. **A** polymeric micelle according to claim 9 wherein:

**Z** is preferably an amide bond;
$R_1$ and $R_2$ are identical and selected from poly(ethylene glycol) or oligo(ethylene glycol) chains, they are preferably triethylene glycol;
when **X** is an anionic group it is preferably selected from carboxylic acids and their derivatives, sulfates and their derivatives, sulfonates and their derivatives,
phosphates and their derivatives, phosphonates and their derivatives, arylphosphonic acids and their derivatives, phenols and their derivatives;
when **X** is a cationic group it is preferably selected from amines, polyamines, oligoamines, preferably spermidines, spermines, anilines, benzylamines, imidazoles, morpholines, ammonium salts, primary, secondary or tertiary amines or guanidinium groups.

11. A polymeric micelle according to claim 1 **characterized in that P** is PEG, **D** contains benozoate groups at its periphery and A is lysozyme.

12. A polymeric micelle according to claim 1 **characterized in that P** is PEG, **D** contains benozoate groups at its periphery and **A** is myoglobin.

13. A polymeric micelle according to claim 1 **characterized in that P** is PEG, **D** contains positively charged aniline groups at its periphery and **A** is insulin.

14. A polymeric micelle according to the previous claims **characterized in that** a drug, diagnostic agent, molecule or macromolecule of any nature is encapsulated in its interior.

**15.** Use of a micelle according to claims 1 to 14 as a carrier for the therapeutic administration of a bioactive protein.

**16.** Use of a micelle according to claims 1 to 14 as a carrier for administering of a drug or diagnostic agent, molecule or macromolecule of any nature.

**17.** A pharmaceutical composition comprising a micelle according to claims 1 to 14 and an active ingredient.

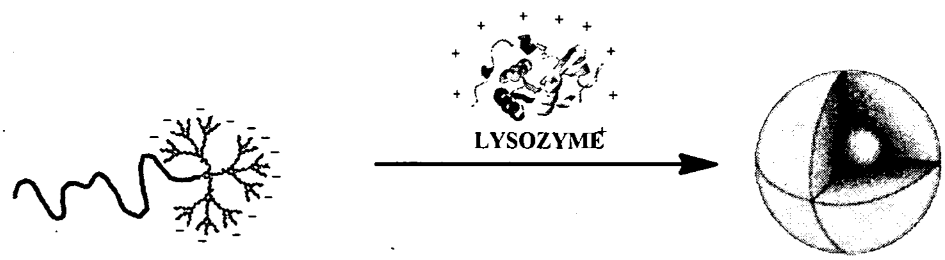


Figure 1

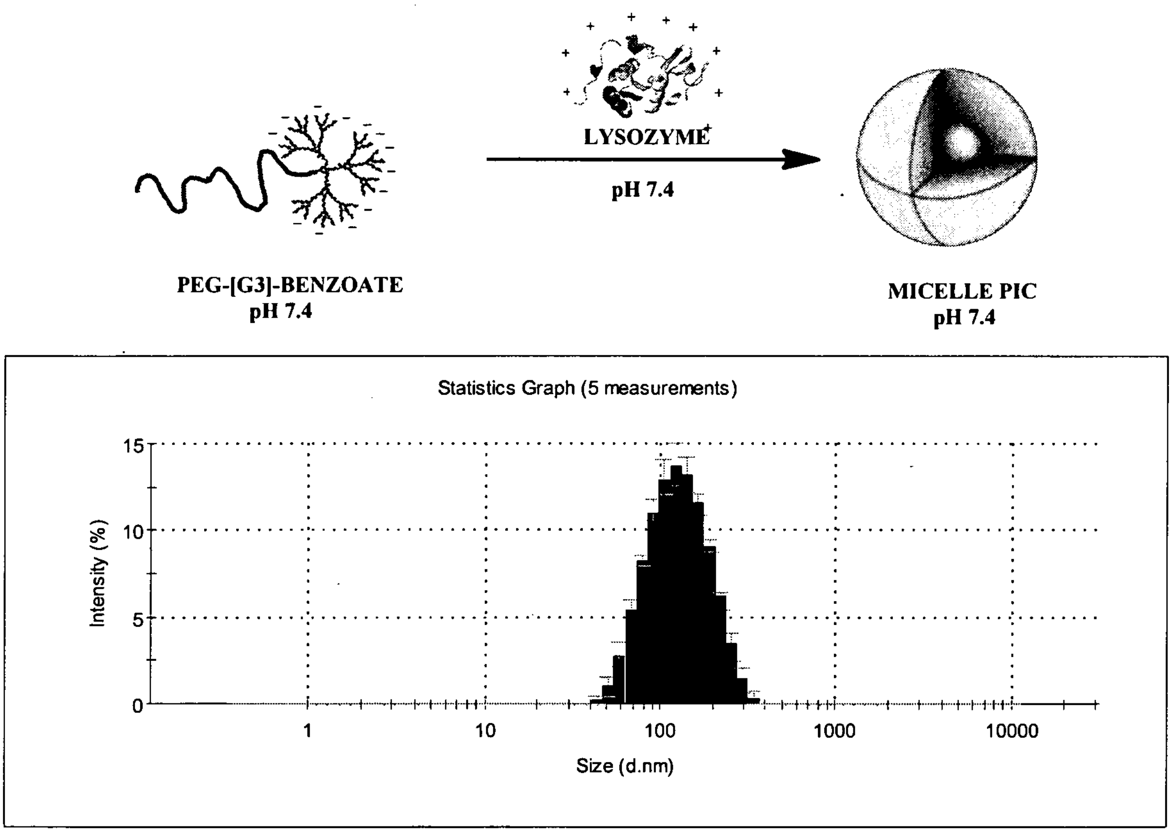


Figure 2

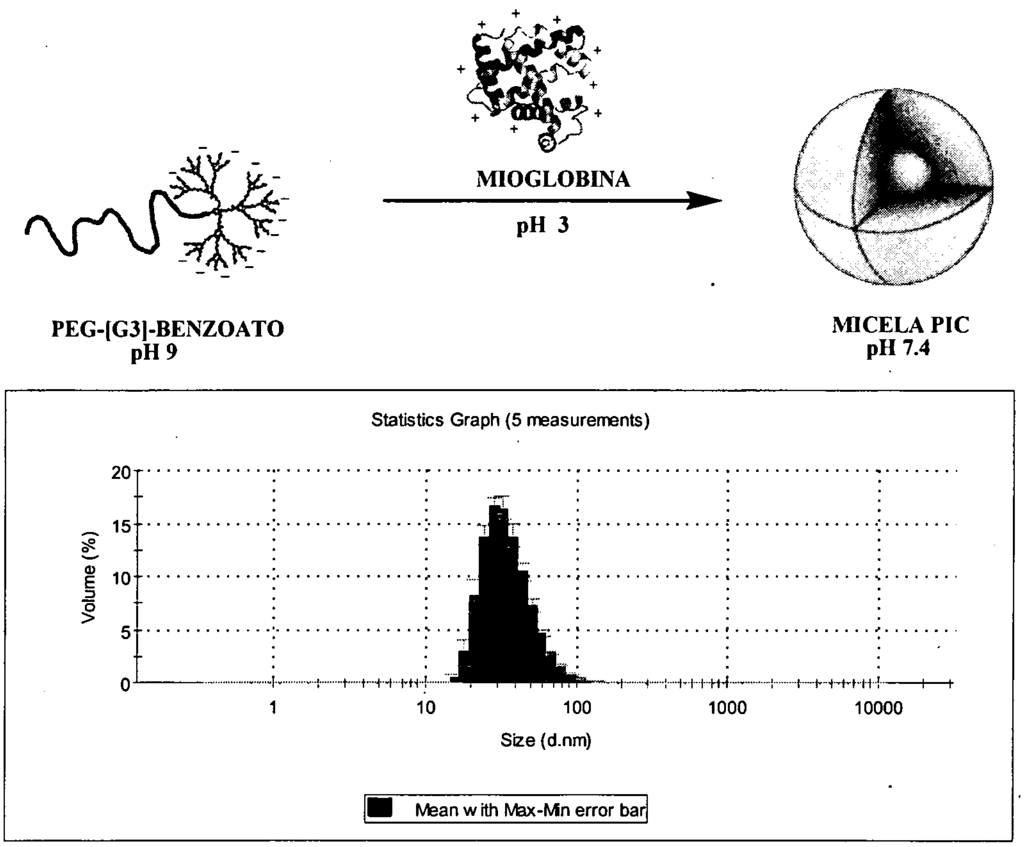
MIOGLOBINA
pH 3
PEG-[G3]-BENZOATO
pH 9
MICELA PIC
pH 7.4
Statistics Graph (5 measurements)
Volume (%)
0
5
10
15
20
1
10
100
1000
10000
Size (d.nm)
Mean with Max-Min error bar

Figure 3

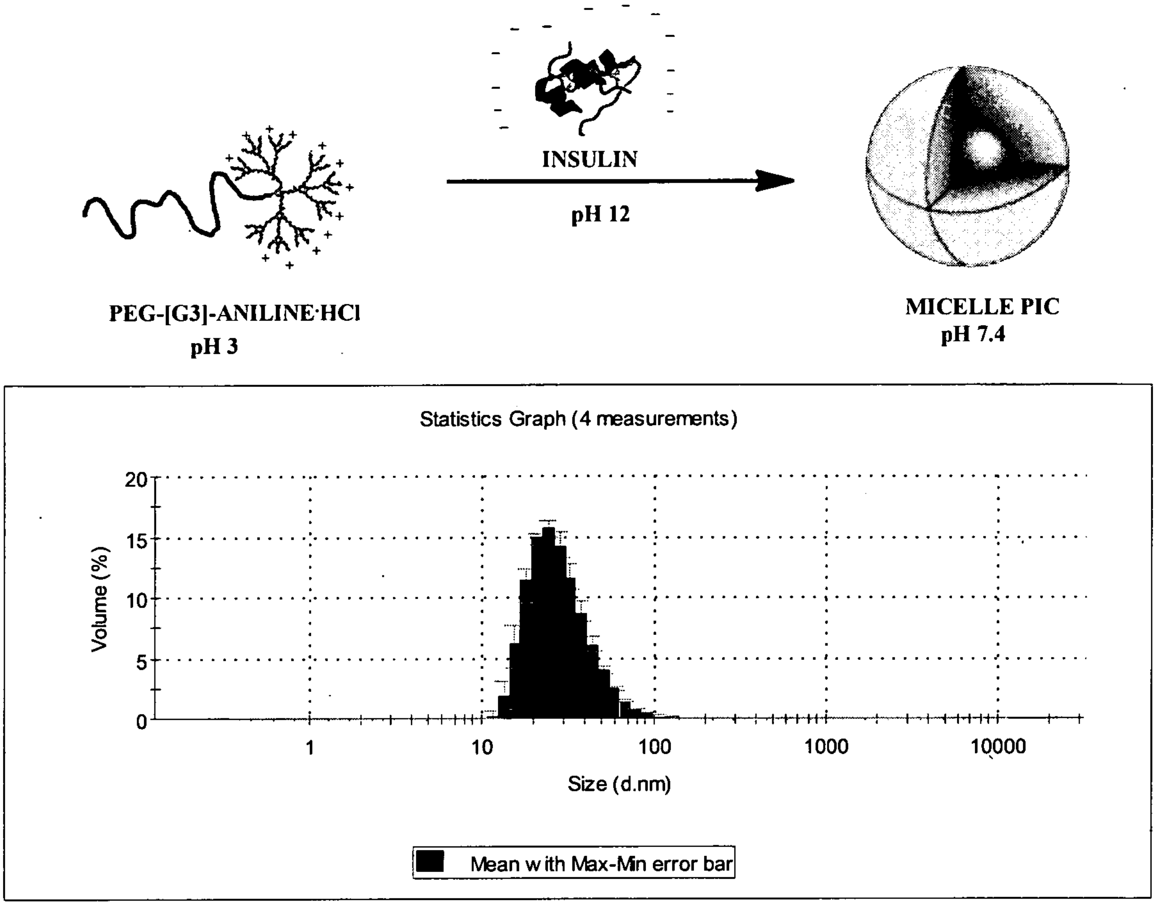
INSULIN
pH 12
PEG-[G3]-ANILINE·HCl
pH 3
MICELLE PIC
pH 7.4
Statistics Graph (4 measurements)
Volume (%)
20
15
10
5
0
1
10
100
1000
10000
Size (d.nm)
Mean with Max-Min error bar

Figure 4

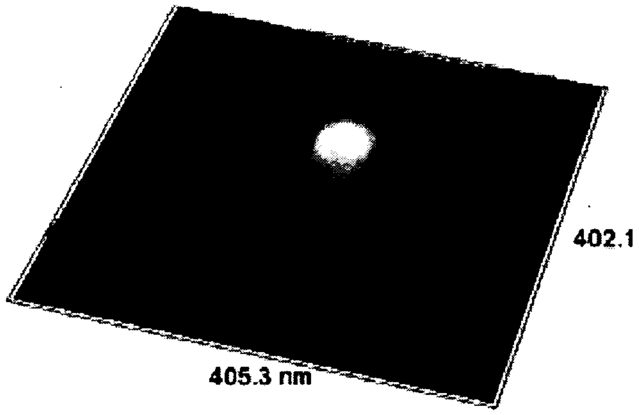


Figure 5

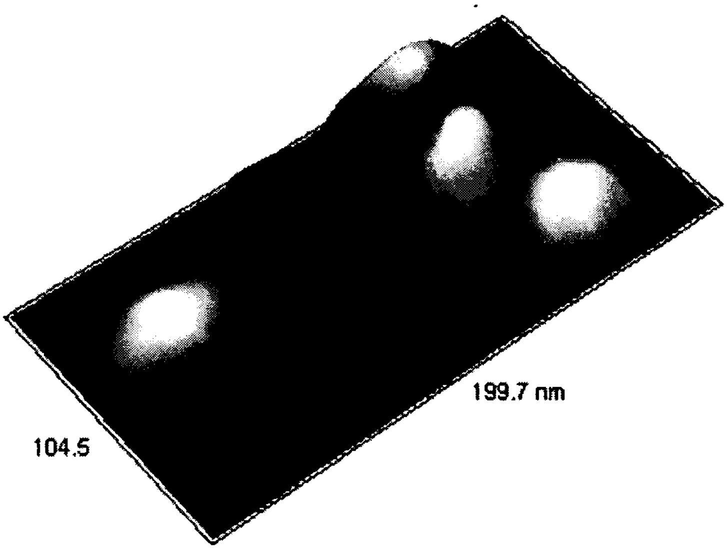


Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Enzymes as Drugs. J. Wiley, 1981, 167
- *J. Polym. Sci. Part A: Polym. Chem.,* 2008, vol. 46, 1
- *Pharm. Res.,* 2001, vol. 18, 1411
- *Macromolecules,* 1995, vol. 28, 5294
- *Bioconjugate Chem.,* 1995, vol. 6, 639
- *Cancer Res.,* 1986, vol. 46, 6387
- *Macromolecules,* 1998, vol. 31, 288
- *J. Am. Chem. Soc.,* 2003, vol. 125, 15306
- *Angew. Chem. Int. Ed.,* 2009
- *Macromolecules,* 2003, vol. 36, 1304
- *Chem. Commun.,* 2008, vol. 27, 3136
- *Chem. Comm.,* 2008, vol. 27, 3136